# FASCICULE DE BREVET EUROPEEN

(11) **EP 3 058 342 B1**
(45) Date de publication et mention de la délivrance du brevet: **08.02.2023**
(21) Numéro de dépôt: 14796829.1
(22) Date de dépôt: 17.10.2014
(51) Int. Cl.: G01N 21/3563, G01N 21/84, G01N 21/88, G01N 21/95, G01N 25/72, G01N 33/32, G01J 3/28

(54) **PROCÉDÉ D'ÉVALUATION DE L'ENDOMMAGEMENT D'UN MATÉRIAU COMPOSITE RECOUVERT D'UNE PEINTURE, MESURANT SUR LE SPECTROGRAMME DEUX CRITÈRES DISTINCTS**
VERFAHREN ZUR BEURTEILUNG VON SCHÄDEN EINES VERBUNDMATERIALS BESCHICHTET MIT FARBE, MESSUNG VON ZWEI VERSCHIEDENEN KRITERIEN IM SPEKTROGRAMM
METHOD OF ASSESSING DAMAGE OF A COMPOSITE MATERIAL COVERED WITH PAINT, MEASURING IN THE SPECTROGRAM TWO SEPARATE CRITERIA

(30) Priorité: 17.10.2013 FR 1360123
(43) Date de publication de la demande: 24.08.2016
(73) Titulaire: Safran Nacelles, 76700 Gonfreville-l'Orcher (FR)
(72) Inventeur: BAILLARD, André, F-76110 Bretteville du Grand Caux (FR); LEFEU, Odile, F-76430 La Remuee (FR); JOUBERT, Frédéric, F-76000 Le Havre (FR); PIEL, Emmanuel, F-76620 Le Havre (FR)
(74) Mandataire: Germain Maureau
(86) Numéro de dépôt international: PCT/FR2014/052656
(87) Numéro de publication internationale: WO 2015/055968

(56) Documents cités:
- FR-A1- 2 981 157
- US-A- 4 492 867
- US-A1- 2011 001 047
- US-A1- 2011 199 098
- US-B1- 6 184 528

## Description

La présente invention concerne un procédé d'évaluation de l'endommagement de matériaux composites recouverts d'une peinture de type polyuréthane.

Les matériaux composites, en particulier les panneaux utilisés dans les applications aéronautiques, à proximité des motorisations, sont prévus pour un fonctionnement à des températures inférieures à un seuil maximum. Ces panneaux formant une couche unique, ou utilisés dans une structure sandwich comprenant une âme centrale comme un nid d'abeille, recouverte par deux peaux extérieures, comportent des caractéristiques mécaniques intéressantes et une masse réduite, et peuvent prendre des formes variées.

Cependant suite à des événements thermiques comprenant une augmentation de la température au-dessus d'un seuil maximum, et suivant les temps d'exposition, on obtient un certain endommagement des matériaux qui peut réduire les caractéristiques de tenue structurale. Il est alors important lors des opérations de maintenance, de pouvoir caractériser le niveau d'endommagement du matériau pour assurer la sécurité.

Afin de réaliser un contrôle sur ces matériaux composites, il est connu d'appliquer une peinture primaire sur la surface, en particulier une peinture polyuréthane, qui va subir des transformations de couleur au cours des événements thermiques.

Un premier procédé connu d'évaluation de l'endommagement, consiste à étudier visuellement la décoloration de la peinture primaire afin d'en déduire un certain niveau d'évolution du matériau. Ce procédé donne une indication qualitative, mais ne permet par une mesure quantitative précise de l'endommagement.

Un autre procédé non destructif connu, présenté notamment par les documents EP-A1-2343531, US-A1-20110001047, ou encore US-B1-6184528 effectue une analyse de la décoloration d'une peinture primaire par une méthode basée sur la spectrométrie infrarouge.

En particulier le deuxième document cité présente le procédé suivant. Après avoir réalisé un modèle d'étalonnage, on émet sur un point de la surface du matériau à analyser un rayonnement infrarouge, puis on mesure le spectre de la réflexion diffuse de cette surface, et en prenant sur ce spectre un critère qui est comparé au modèle d'étalonnage, on évalue un historique des aspects thermiques ou chimiques du revêtement, pour en déduire un état du matériau composite le supportant.

Toutefois ce procédé est complexe à réaliser, et les résultats obtenus dépendent fortement de la mise en oeuvre de la peinture primaire. En particulier des variations d'épaisseur de la couche du revêtement, de son cycle de polymérisation, du nombre de couches déposées ou de l'état de surface, vont perturber la réflexion des infrarouges. Les résultats ainsi que les conclusions relatives à l'endommagement du matériau, ne sont pas précis.

D'autres procédés non destructifs connus d'évaluation de l'endommagement des matériaux, utilisent des méthodes classiques par ultrason ou thermographie notamment, qui permettent en particulier de détecter des décollements entre les couches des matériaux. On peut ainsi contrôler par exemple le décollement entre la peau et l'âme d'un panneau comprenant une structure sandwich. Toutefois ce procédé ne permet pas d'évaluer l'état du matériau par lui-même.

La présente invention a notamment pour but d'éviter ces inconvénients de la technique antérieure.

Elle propose à cet effet un procédé d'évaluation de l'endommagement d'un matériau composite recouvert d'une peinture du type polyuréthane, utilisant une spectrométrie infrarouge de cette peinture, remarquable en ce qu'on mesure sur le même spectrogramme obtenu deux critères distincts caractérisant le vieillissement thermique de la peinture, chacun desdits deux critères distincts comprenant la mesure sur la courbe du spectrogramme d'une hauteur d'un pic particulier, donnant deux évaluations indépendantes de ce vieillissement thermique de la peinture, puis on combine ces deux critères distincts entre eux pour obtenir un résultat significatif du niveau d'endommagement.

Un avantage de ce procédé d'évaluation est que de manière rapide et économique, la comparaison simultanée des deux critères combinés permet de s'affranchir des variations des paramètres de mise en oeuvre de la peinture, et des conditions de mesure, ce qui donne une évaluation précise de l'endommagement du matériau.

Dans le cadre des revendications annexées, le procédé d'évaluation de l'endommagement selon l'invention peut comporter de plus une ou plusieurs des caractéristiques suivantes, qui peuvent être combinées entre elles.

Avantageusement pour obtenir un premier critère, la hauteur du pic particulier est mesurée par rapport à une ligne de base passant par les deux premiers creux immédiatement adjacents de chaque côté de ce pic. Cette mesure est simple à réaliser.

Avantageusement pour obtenir un deuxième critère, la hauteur du pic particulier est mesurée par rapport à une ligne de base passant par les deux premiers creux importants de chaque côté de ce pic. De la même manière, cette mesure est simple à réaliser.

Avantageusement, on dispose les deux critères suivant les deux axes d'un graphique afin de les combiner, ce graphique comprenant des zones séparées par des limites, qui déterminent le niveau de dégradation thermique du matériau. On réalise ainsi de manière simple et rapide une évaluation de l'endommagement.

En particulier, le graphique peut comporter une première zone correspondant à une absence d'évolution de la structure chimique du matériau, une deuxième zone correspondant à une évolution de cette structure entraînant un faible endommagement, et une troisième zone correspondant à une évolution de cette structure entraînant un fort endommagement.

La limite entre la première zone et la deuxième, peut correspondre à un vieillissement équivalent particulier (température / durée). La limite entre la deuxième zone et la troisième, peut correspondre à un autre vieillissement équivalent particulier. Ces valeurs sont représentatives de seuils de transformation permettant d'établir la catégorie de l'endommagement.

Selon un mode de réalisation du procédé, la peinture polyuréthane de référence 5014 est utilisée.

Suivant d'autres caractéristiques optionnelles du procédé selon l'invention :
- le nombre d'onde du pic particulier est proche de 1300 cm⁻¹, étant compris entre 1288 cm⁻¹ et 1314 cm⁻¹ ;
- pour obtenir le premier critère, on prend le premier creux compris entre 1322 cm⁻¹ et 1374 cm⁻¹, et le deuxième creux compris entre 1236 cm⁻¹ et 1247 cm⁻¹, la valeur de 1247 cm⁻¹ étant retenue si ce deuxième creux n'est pas prononcé sur la courbe ;
- dans le cas où le pic particulier n'est plus observable, on mesure alors entre 1288 cm⁻¹ et 1314 cm⁻¹ la plus grande hauteur comprise entre la ligne de base et la courbe, pour obtenir le premier critère qui peut être négatif ;
- pour obtenir le deuxième critère, on prend le premier creux important proche de 2000 cm⁻¹, compris entre 1951 cm⁻¹ et 2051 cm⁻¹, et le deuxième creux important proche de 1000 cm⁻¹, compris entre 901 cm⁻¹ et 1102 cm⁻¹ ;
- dans le cas où le pic particulier n'est plus observable, on mesure alors la hauteur entre la ligne de base et la courbe pour la valeur 1288 cm⁻¹, pour obtenir le deuxième critère.

L'invention sera mieux comprise et d'autres caractéristiques et avantages apparaîtront plus clairement à la lecture de la description ci-après donnée à titre d'exemple, en référence aux dessins annexés dans lesquels :
- la figure 1 est un spectrogramme infrarouge obtenu pour l'analyse d'un matériau composite avec un procédé selon un mode de réalisation l'invention ;
- la figure 2 est une vue de détail de ce spectrogramme montrant la mesure du premier critère de ce procédé, pour un pic particulier nettement visible ;
- les figures 3 et 4 présentent une méthode de mesure du premier critère pour un pic particulier faiblement visible ;
- les figures 5 et 6 présentent une méthode de mesure du premier critère pour un pic particulier non visible ;
- la figure 7 présente en fonction du temps d'exposition du matériau et de la température atteinte, la valeur du premier critère ;
- la figure 8 présente une méthode de mesure du deuxième critère ;
- la figure 9 est un graphique présentant en fonction des deux critères, le principe de variation de l'exposition thermique ; et
- la figure 10 est le même graphique montrant pour des expositions thermiques identiques, des mesures pour des peintures comportant différentes conditions de mise en oeuvre.

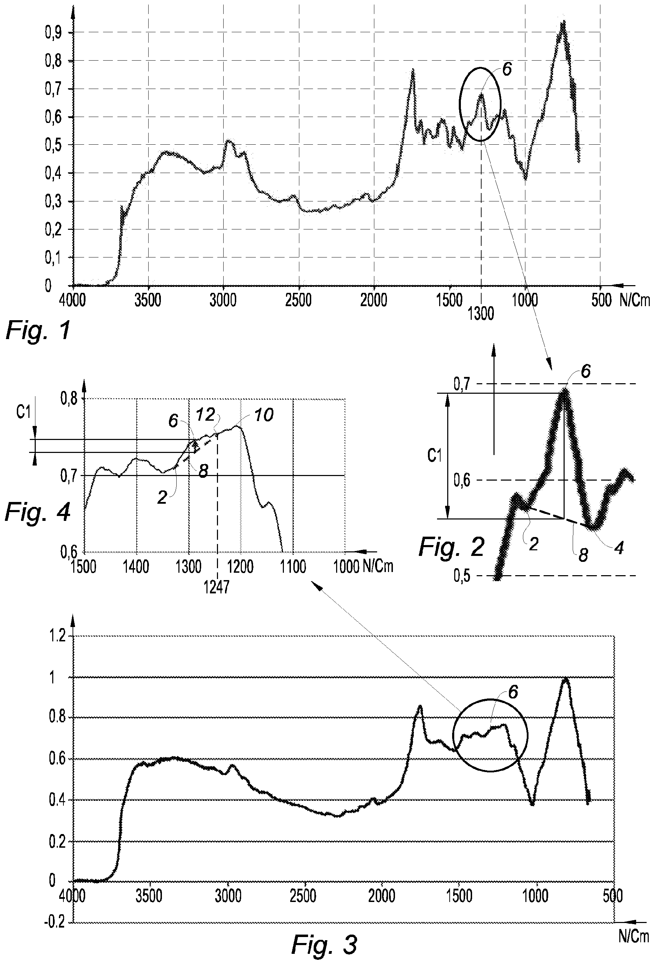

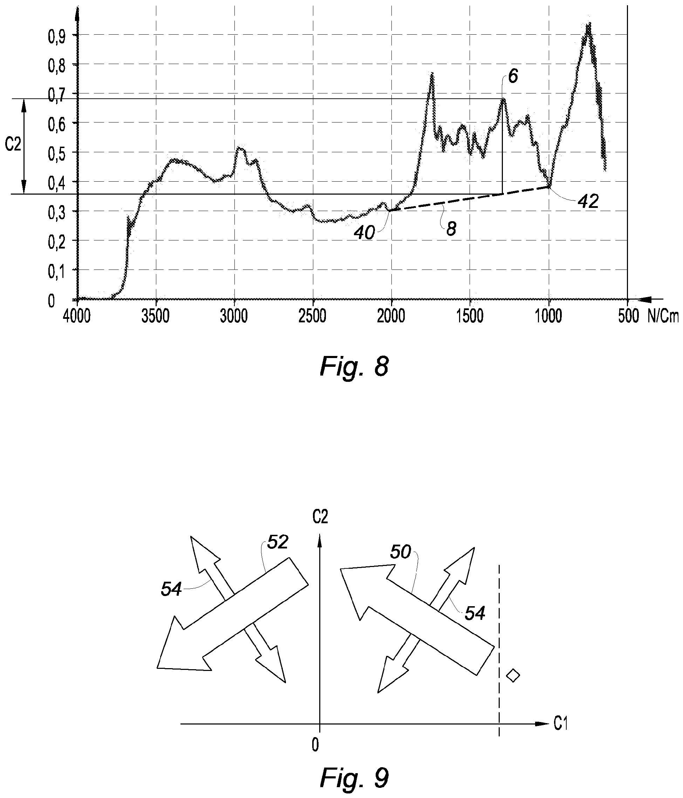

Les figures 1 et 8 présentent en fonction du nombre d'onde par centimètre N/cm, la courbe obtenue pour une spectrométrie sur un panneau en matériaux composites recouvert d'une peinture primaire polyuréthane de type 5014. La mesure est effectuée pour un nombre d'onde compris entre 4000 cm⁻¹ et 650 cm⁻¹.

On peut en particulier effectuer cette analyse sur des pièces en fibre de carbone et en résine du type bismaleimide « BMI », recouverte par le primaire polyuréthane.

Le spectre obtenu est caractéristique d'une peinture primaire polyuréthane avec cette référence, il évolue en fonction de l'exposition à la température, comprenant le niveau de température et la durée d'exposition.

La spectrométrie est réalisée avec un appareil portatif mis en contact avec la pièce, selon la technique de réflexion diffuse, adaptée à l'état de surface du panneau en matériaux composites. L'état de surface de la pièce et au préalable préparé par un nettoyage avec un solvant, pour éliminer la pollution qui pourrait perturber les mesures et modifier le spectrogramme.

A partir de ce spectrogramme, deux critères différents C1 et C2 sont identifiés sur la courbe pour caractériser le vieillissement thermique.

L'utilisation de deux critères permet d'obtenir par une comparaison de ces critères, une évaluation du vieillissement thermique qui est indépendante des paramètres de mise en oeuvre de la peinture polyuréthane et des conditions de mesure, ce qui fiabilise la mesure. On notera que l'utilisation d'un unique critère ne permet pas d'effectuer de comparaison, on reste tributaire des paramètres de mise en oeuvre et des conditions de mesure, ce qui ne donne pas de résultat fiable.

Pour le calcul du premier critère C1 présenté figure 1, on mesure sur le spectrogramme la hauteur d'un pic particulier 6 par rapport à une ligne de base 8 passant par les deux premiers creux immédiatement adjacents 2, 4 de chaque côté de ce pic. Pour le calcul du deuxième critère C2 présenté figure 8, on mesure la hauteur du pic particulier 6 par rapport à une ligne de base 8 passant par les deux premiers creux importants 40, 42 de chaque côté de ce pic.

On notera que les creux sur la courbe représentant des points de faible émission qui varient peu au cours du vieillissement thermique, constituent des références permettant de mesurer l'évolution de la hauteur du pic 6.

Dans cet exemple avec une peinture primaire polyuréthane de type 5014, le pic particulier 6 est choisi proche du nombre d'onde de 1300 cm⁻¹, étant compris entre 1288 cm⁻¹ et 1314 cm⁻¹.

Les figures 1 et 2 présentent le cas d'un spectrogramme disposant du pic particulier 6 observable facilement grâce à un premier point 2 correspondant au creux situé juste à gauche, et à un deuxième point 4 correspondant au creux situé juste à droite. On mesure alors la hauteur de ce pic 6 par rapport à la ligne de base 8 reliant les deux points 2, 4, pour obtenir le premier critère C1.

Les figures 3 et 4 présentent le cas d'un spectrogramme disposant d'un pic particulier 6 qui est réduit par la présence d'un deuxième pic 10 situé à droite, à environ 1200 cm⁻¹, avec une absence du deuxième creux entre le pic particulier 6 et ce deuxième pic 10.

Dans ce cas on dispose une ligne de base 8 qui passe par le premier point 2 correspondant au creux gauche 2, et par un deuxième point de la courbe 12 pris pour la valeur 1247 cm⁻¹. On mesure de la même manière la hauteur du pic particulier 6 par rapport à cette ligne de base 8, pour obtenir le deuxième critère C2.

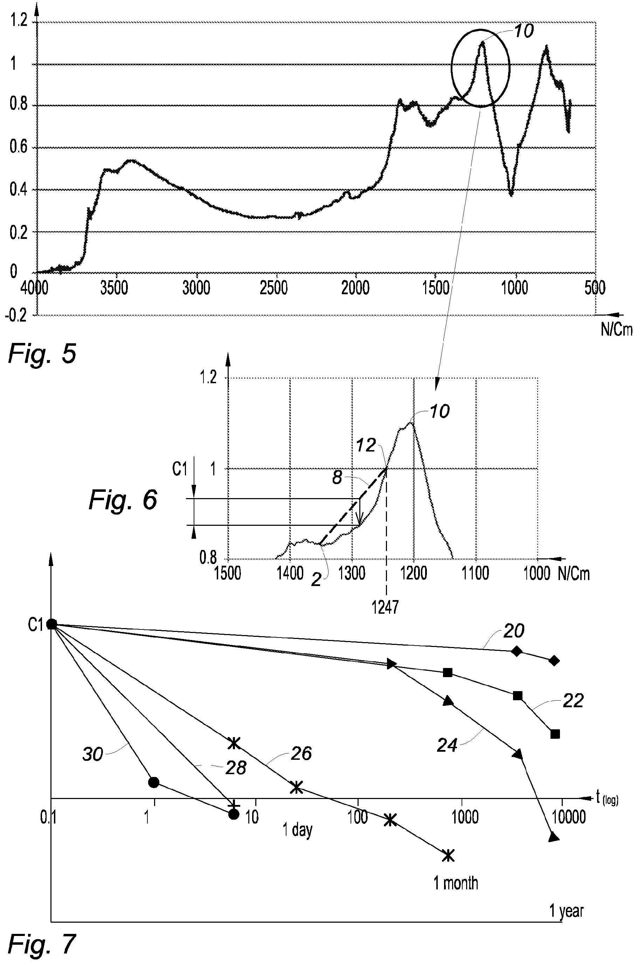

Les figures 5 et 6 présentent le cas d'un spectrogramme disposant d'un pic particulier 6 qui n'est plus observable à cause de la présence du deuxième pic 10 situé à droite, à environ 1200 cm⁻¹, qui est très développé. On a alors une absence totale de creux vers 1250 cm⁻¹.

Dans ce cas on dispose aussi une ligne de base 8 qui passe par le premier point 2 correspondant au creux gauche, et par le deuxième point de la courbe 12 pris pour la valeur 1247 cm⁻¹. On mesure ensuite la plus grande hauteur entre cette ligne de base 8 et la courbe, comprise entre 1288 cm⁻¹ et 1314 cm⁻¹, pour obtenir le premier critère C1. Dans cet exemple la hauteur est négative, ce premier critère C1 est alors négatif.

D'une manière générale pour les calculs du premier critère C1, la ligne de base 8 passe par un premier point correspondant au creux gauche 2 compris entre 1322 cm⁻¹ et 1374 cm⁻¹, et par un deuxième point 4 correspondant au creux droit compris entre 1236 cm⁻¹ et 1247 cm⁻¹, ou en l'absence de creux droit par un deuxième point 12 pour la valeur de 1247 cm⁻¹.

La figure 7 présente en fonction du temps d'exposition indiqué sur une échelle logarithmique, la valeur du premier critère C1 présentée sur des courbes référencées 20, 22, 24, 26, 28, 30, correspondant respectivement à des températures de 100, 150, 170, 200, 250 et 300°C.

On constate que ce premier critère C1 est bien caractéristique du vieillissement thermique, avec une variation dépendant à la fois du niveau de température et du temps d'exposition.

La figure 8 présente le calcul du deuxième critère C2, qui mesure la hauteur du pic particulier 6 par rapport à une ligne de base 8 reliant le premier creux important 40 situé à gauche vers 2000 cm⁻¹, compris entre 1951 cm⁻¹ et 2051 cm⁻¹, au deuxième creux important 42 situé à droite vers 1000 cm⁻¹, compris entre 901 cm⁻¹ et 1102 cm⁻¹.

Dans le cas où le pic particulier 6 n'est plus observable par la présence d'un deuxième pic situé à droite à environ 1200 cm⁻¹ qui est très développé, comme le présentent les figures 5 et 6, on mesure alors la hauteur entre la ligne de base 8 et la courbe pour la valeur 1288 cm⁻¹, afin d'obtenir la mesure du deuxième critère C2.

La figure 9 présente un graphique comprenant sur un axe horizontal le premier critère C1 et sur un axe vertical le deuxième critère C2, sur lequel on dispose les critères mesurés du matériau à analyser.

Les flèches principales 50 puis 52 présentent en suivant leur parcours une modification des deux critères C1, C2 donnant une exposition thermique de plus en plus importante, par le niveau de température atteint plus élevé ou le temps d'exposition plus long, qui va donner une dégradation de plus en plus forte du matériau.

Par contre les flèches transversales 54 présentent suivant leurs directions une modification des deux critères C1, C2 correspondant à une variation des paramètres de mise en oeuvre de la peinture polyuréthane, ou des états de surface, ne déplaçant pas les points suivant la direction des flèches principales 50, 52, qui donne une dégradation thermique similaire du matériau.

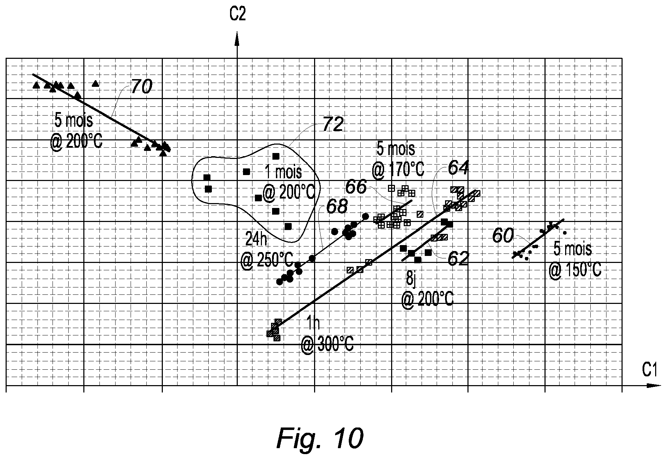

La figure 10 présente sur le même graphique différentes mesures qui viennent confirmer le principe présenté ci-dessus. Chaque lot de mesures regroupe sensiblement autour d'un axe de pente constante, aligné suivant les directions des flèches transversales 54, un ensemble de points correspondant au même vieillissement thermique, sur une peinture polyuréthane mise en oeuvre dans des conditions différentes.

On a en particulier un premier axe 60 correspondant à une exposition à la température de 150° pendant cinq mois, un deuxième axe 62 correspondant à 200° pendant huit jours, un troisième axe 64 correspondant à 300° pendant une heure, un quatrième axe 66 correspondant à 170° pendant cinq mois, un cinquième axe 68 correspondant à 250° pendant vingt-quatre heures, et un sixième axe 70 correspondant à 200° pendant cinq mois. On a aussi un groupe de points 72 correspondant à 200° pendant un mois, qui se trouve à proximité du premier critère C1 égal à zéro.

On obtient ainsi de manière simple et rapide, sans destruction du matériau, une évaluation précise du niveau d'engagement thermique des matériaux, qui est indépendante des paramètres de la mise en oeuvre de la peinture primaire, et des conditions de mesure.

## Revendications

1. - Procédé d'évaluation de l'endommagement d'un matériau composite recouvert d'une peinture du type polyuréthane, dans lequel on mesure sur un même spectrogramme obtenu par spectrométrie infrarouge deux critères distincts (C1, C2) caractérisant le vieillissement thermique de la peinture, chacun desdits deux critères distincts comprenant la mesure sur la courbe du spectrogramme d'une hauteur d'un pic particulier (6), donnant deux évaluations indépendantes de ce vieillissement thermique de la peinture, puis on combine ces deux critères distincts (C1, C2) entre eux pour obtenir un résultat significatif du niveau d'endommagement.

2. - Procédé d'évaluation selon la revendication 1, dans lequel pour obtenir un premier critère (C1), la hauteur du pic particulier (6) est mesurée par rapport à une ligne de base (8) passant par les deux premiers creux immédiatement adjacents (2, 4) de chaque côté de ce pic.

3. - Procédé d'évaluation selon la revendication 1 ou 2, dans lequel pour obtenir un deuxième critère (C2), la hauteur du pic particulier (6) est mesurée par rapport à une ligne de base (8) passant par les deux premiers creux importants (40, 42) de chaque côté de ce pic.

4. - Procédé d'évaluation selon l'une quelconque des revendications précédentes, dans lequel on dispose les deux critères (C1, C2) suivant les deux axes d'un graphique afin de les combiner, et de déterminer le niveau de dégradation thermique du matériau.

5. - Procédé selon l'une quelconque des revendications précédentes, dans lequel le nombre d'onde du pic particulier (6) est proche de 1300 cm⁻¹, étant compris entre 1288 cm⁻¹ et 1314 cm⁻¹.

6. - Procédé d'évaluation selon les revendications 2 et 5, dans lequel pour obtenir le premier critère (C1), on prend le premier creux (2) compris entre 1322 cm⁻¹ et 1374 cm⁻¹, et le deuxième creux (4) compris entre 1236 cm⁻¹ et 1247 cm⁻¹, la valeur de 1247 cm⁻¹ étant retenue si ce deuxième creux n'est pas prononcé sur la courbe.

7. - Procédé d'évaluation selon les revendication 2 et 5, dans lequel dans le cas où le pic particulier (6) n'est plus observable, on mesure alors entre 1288 cm⁻¹ et 1314 cm⁻¹ la plus grande hauteur comprise entre la ligne de base (8) et la courbe, pour obtenir le premier critère (C1) qui peut être négatif.

8. - Procédé d'évaluation selon les revendications 3 et 5, dans lequel pour obtenir le deuxième critère (C2), on prend le premier creux important (40) proche de 2000 cm⁻¹, compris entre 1951 cm⁻¹ et 2051 cm⁻¹, et le deuxième creux important (42) proche de 1000 cm⁻¹, compris entre 901 cm⁻¹ et 1102 cm⁻¹.

9. - Procédé d'évaluation selon la revendication 8, dans lequel dans le cas où le pic particulier (6) n'est plus observable, on mesure alors la hauteur entre la ligne de base (8) et la courbe pour la valeur 1288 cm⁻¹, pour obtenir le deuxième critère (C2).

## Patentansprüche

1. Verfahren zur Beurteilung von Schäden eines Verbundmaterials, das mit einer Farbe vom Typ Polyurethan beschichtet ist, wobei man auf einem einzelnen Spektrogramm, das durch Infrarot-Spektrometrie erhalten wird, zwei unterschiedliche Kriterien (C1, C2) misst, die die thermische Alterung der Farbe charakterisieren, wobei jedes dieser beiden unterschiedlichen Kriterien die Messung auf der Kurve des Spektrogramms einer Höhe einer bestimmten Spitze (6) umfasst, zwei unabhängige Bewertungen dieser thermischen Alterung der Farbe ergebend, dann kombiniert man diese beiden unterschiedlichen Kriterien (C1, C2) untereinander, um ein signifikantes Ergebnis der Ebene des Schadens zu erhalten.

2. Verfahren zur Beurteilung nach Anspruch 1, in welchem, um ein erster Kriterium (C1) zu erhalten, die Höhe der bestimmten Spitze (6) gemessen wird in Bezug auf eine Basislinie (8), die die beiden ersten unmittelbar angrenzenden Tiefpunkte (2, 4) auf jeder Seite der Spitze durchläuft.

3. Verfahren zur Beurteilung nach Anspruch 1 oder 2, wobei, um ein zweites Kriterium (C2) zu erhalten, die Höhe der bestimmten Spitze (6) gemessen wird in Bezug auf eine Basislinie (8), die die beiden ersten großen Tiefpunkte (40, 42) auf jeder Seite dieser Spitze durchläuft.

4. Verfahren zur Beurteilung nach einem der vorhergehenden Ansprüche, wobei man die beiden Kriterien (C1, C2) bekommt durch das Verfolgen der beiden Achsen einer Grafik, um sie zu kombinieren und die Ebene der thermischen Degradation des Materials zu bestimmen.

5. Verfahren nach einem der vorhergehenden Ansprüche, wobei die Wellenzahl der bestimmten Spitze (6) nahe 1300 cm ⁻¹, zwischen 1288 cm⁻¹ und 1314 cm⁻¹ liegt.

6. Verfahren zur Beurteilung nach einem der Ansprüche 2 bis 5, wobei man, um das erste Kriterium (C1) zu erhalten, den ersten Tiefpunkt (2) nimmt, der zwischen 1322 cm⁻¹ und 1374 cm⁻¹ liegt, und den zweiten Tiefpunkt (4), der zwischen 1236 cm⁻¹ und 1247 cm⁻¹ liegt, wobei der Wert von 1247 cm⁻¹ zurückgehalten wird, wenn der zweite Tiefpunkt nicht auf der Kurve ausgeprägt ist.

7. Verfahren zur Beurteilung nach den Ansprüchen 2 und 5, wobei man in dem Fall, in dem die bestimmte Spitze (6) nicht mehr beobachtbar ist, zwischen 1288 cm⁻¹ und 1314 cm⁻¹ die größte Höhe misst, die zwischen der Basislinie (8) und der Kurve liegt, um das erste Kriterium (C1) zu erhalten, das negativ sein kann.

8. Verfahren zur Beurteilung nach den Ansprüchen 3 und 5, wobei man, um das zweite Kriterium (C2) zu erhalten, den ersten großen Tiefpunkt (40) nahe 2000 cm⁻¹ nimmt, der zwischen 1951 cm⁻¹ und 2051 cm⁻¹ liegt, und den zweiten großen Tiefpunkt (42) nahe 1000 cm⁻¹, der zwischen 901 cm⁻¹ und 1102 cm⁻¹ liegt.

9. Verfahren zur Beurteilung nach Anspruch 8, wobei man in dem Fall, in dem die bestimmte Spitze (6) nicht mehr beobachtbar ist, die Höhe zwischen der Basislinie (8) und der Kurve für den Wert 1288 cm⁻¹ misst, um das zweite Kriterium (C2) zu erhalten.

## Claims

1. A method for assessing damage to a composite material covered with a polyurethane-type paint in which two separate criteria (C1, C2) characterizing the thermal aging of the paint are measured on the same spectrogram obtained by infrared spectrometry, each of said two separate criteria comprising the measurement on the curve of the spectrogram of a height of a particular peak (6), giving two independent assessments of this thermal aging of the paint, then these two separate criteria (C1, C2) are combined between them to obtain a significant result of the level of damage.

2. The assessment method according to claim 1, wherein in order to obtain a first criterion (C1), the height of the particular peak (6) is measured relative to a base line (8) passing through the two first immediately adjacent troughs (2, 4) on each side of the peak.

3. The assessment method according to claim 1 or 2, wherein in order to obtain a second criterion (C2), the height of the particular peak (6) is measured relative to a base line (8) passing through the two first significant troughs (40, 42) on each side of the peak.

4. The assessment method according to any one of the preceding claims, wherein the two separate criteria (C1, C2) are disposed along two axes of a graph in order to combine them, and to determine a level of thermal degradation of the material.

5. The method according to any one of the preceding claims, wherein the wave number of the particular peak (6) is about 1300 cm⁻¹, being comprised between 1288 cm⁻¹ and 1314 cm⁻¹.

6. The assessment method according to claims 2 and 5, wherein in order to obtain the first criterion (C1), the first trough (2) comprised between 1322 cm⁻¹ and 1374 cm⁻¹ is considered, and the second trough (4) comprised between 1236 cm⁻1 and 1247 cm⁻¹ is considered, the value 1247 cm⁻¹ being retained if this second trough is not pronounced on the curve.

7. The assessment method according to claims 2 and 5, wherein in a case where the particular peak (6) is no longer observable, a largest height comprised between the base line (8) and the curve is measured between 1288 cm⁻¹ and 1314 cm⁻¹, in order to obtain the first criterion (C1) that may be negative.

8. The assessment method according to claims 3 and 5, wherein in order to obtain the second criterion (C2), the first significant trough (40) is about 2000 cm ⁻¹, comprised between 1951 cm⁻¹ and 2051 cm⁻¹, and the second significant trough (42) is about 1000 cm⁻¹, comprised between 901 cm⁻¹ and 1102 cm⁻¹ are considered.

9. The assessment method according to claim 8, wherein in the case where the particular peak (6) is no longer observable, then the height between the base line (8) and the curve at a 1288 cm⁻¹ value is measured, in order to obtain the second criterion (C2).
